(19) Europäisches Patentamt
European Patent Office
Office européen des brevets

(11) Veröffentlichungsnummer: **0 413 224 A1**

# (12) EUROPÄISCHE PATENTANMELDUNG

(21) Anmeldenummer: **90115048.2**

(22) Anmeldetag: **06.08.90**

(51) Int. Cl.[5]: **A01N 35/06**, A01N 37/34, A01N 43/16, A01N 43/40, A01N 37/02, A01N 37/10, A01N 37/22, A01N 37/18, A01N 43/12

(30) Priorität: **12.08.89 DE 3926747**

(43) Veröffentlichungstag der Anmeldung: **20.02.91 Patentblatt 91/08**

(84) Benannte Vertragsstaaten: **AT BE CH DE DK ES FR GB GR IT LI NL SE**

(71) Anmelder: **BASF Aktiengesellschaft**
**Carl-Bosch-Strasse 38**
**D-6700 Ludwigshafen(DE)**

(72) Erfinder: **Ammermann, Eberhard, Dr. Dipl.-Chem.**
**Sachsenstrasse 3**
**D-6700 Ludwigshafen(DE)**
Erfinder: **Lorenz, Gisela, Dr. Dipl.-Ing.**
**Erlenweg 13**
**D-6730 Neustadt(DE)**
Erfinder: **Ditrich, Klaus, Dr. Dipl.-Chem.**
**Paray-Le-Monial-Strasse 12**
**D-6702 Bad Duerkheim(DE)**
Erfinder: **Hamprecht, Gerhard, Dr. Dipl.-Chem.**
**Rote-Turm-Strasse 28**
**D-6940 Weinheim(DE)**

## (54) 1,2-Naphthochinone enthaltende fungizide Mittel.

(57) Fungizide Mittel, enthaltend inerte Trägerstoffe und ein 1,2-Naphthochinon der Formel I

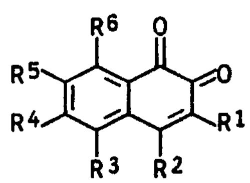

I

($R^1$-$R^6$ = H, Halogen oder 3 Reste = CN, $NO_2$, $C_1$-$C_{10}$-Halogenalkyl, $C_2$-$C_{10}$-Halogenalkenyl, $C_2$-$C_{10}$-Alkinyl, $C_3$-$C_6$-Cycloalkyl, $C_3$-$C_6$-Alkoxy-alkoxycarbonyl, $C_1$-$C_{10}$-Alkyl oder $C_2$-$C_{10}$-Alkenyl, die beide weitere Substituenten tragen können; oder 3 Reste = $XR^7$; X = O, S, -CO-, -CO-O-, -O-CO-, -$NR^8$-CO-, -CO-$NR^8$-, $C_1$-$C_4$-CO-$NR^8$; $R^7$, $R^8$ = H, $C_1$-$C_5$-Alkyl, $C_1$-$C_5$-Halogenalkyl, $C_3$-$C_6$-Alkenyl, $C_3$-$C_6$-Alkinyl; oder 2 Reste = Aziridinyl, Pyrrolidino, Piperidino oder Morpholino, wobei die beiden letzten noch 2 weitere Substituenten tragen können; oder 2 Reste = Phenyl, Benzyl oder $C_5$-$C_6$-Heteroaryl, an das ein Benzolring annelliert sein kann und wobei alle Arylteile bis zu 3 weitere Substituenten tragen können; oder $R^2$, $R^6$ = Reste $YR^9$; Y = -O-CO-, -$NR^8$-; $R^9$ = Phenyl, Benzyl, die weitere Substituenten tragen können; oder $R^2$, $R^6$ = eine Gruppe -($NR^{10}$-$SO_2R^{11}$) oder -(O-$SO_2R^{11}$); $R^{10}$ = H, $C_1$-$C_{10}$-Alkyl, $C_1$-$C_{10}$-Halogenalkyl, $C_3$-$C_{10}$-Alkenyl, $C_3$-$C_{10}$-Alkinyl, $C_2$-$C_{14}$-Alkoxyalkyl, $C_2$-$C_{14}$-Alkylthioalkyl, $C_3$-$C_7$-Cycloalkyl; $R^{11}$ = $C_1$-$C_{10}$-Alkyl, $C_2$-$C_8$-Alkenyl, Trifluormethyl, Cyclopropyl oder Phenyl, das bis zu 3 weitere Substituenten tragen kann; oder 2 benachbarte Substituenten bedeuten zusammen einen annellierten 5- oder 6-gliedrigen ungesättigten Ring, an den zusätzlich ein Benzolring annelliert sein kann und der S, 1-2 nicht benachbarte O-Atome oder 1-3 N-Atome als Heteroatome enthalten und bis zu 2 weitere Substituenten tragen kann).

EP 0 413 224 A1

# 1,2-NAPHTHOCHINONE ENTHALTENDE FUNGIZIDE MITTEL

Die vorliegende Erfindung betrifft fungizide Mittel, enthaltend inerte Trägerstoffe und ein 1,2-Naphthochinon der allgemeinen Formel I

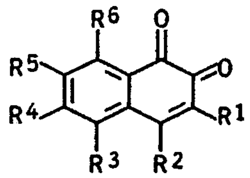

I

in der die Substituenten $R^1$ bis $R^6$ die nachstehende Bedeutung haben:

a) Wasserstoff oder Halogen;

b) bis zu 3 der Substituenten $R^1$ bis $R^6$ die folgenden Gruppen:
Cyan, Nitro, partiell oder vollständig halogeniertes $C_1$-$C_{10}$-Alkyl, $C_2$-$C_{10}$-Alkenyl mit bis zu 2 Halogensubstituenten, $C_2$-$C_{10}$-Alkinyl, $C_3$-$C_6$-Cycloalkyl, $C_3$-$C_6$-Alkoxy-alkoxycarbonyl, $C_1$-$C_{10}$-Alkyl oder $C_2$-$C_{10}$-Alkenyl, wobei die letzten beiden Gruppen bis zu zwei der folgenden Substituenten tragen können: Hydroxy, Cyano, $C_1$-$C_4$-Alkoxy, $C_1$-$C_4$-Alkylthio oder $C_1$-$C_4$-Alkoxycarbonyl;

c) bis zu 3 der Substituenten $R^1$ bis $R^6$ eine Gruppe der allgemeinen Formel:
$XR^7$, wobei X Sauerstoff, Schwefel, eine Gruppe -CO-, -CO-O-, -O-CO-, $-NR^8-$, $-NR^8-CO-$, $-CO-NR^8-$ oder $C_1$-$C_4$-$CO-NR^8$ und $R^7$ und $R^8$ Wasserstoff, $C_1$-$C_5$-Alkyl, das partiell oder vollständig halogeniert sein kann, $C_3$-$C_6$-Alkenyl oder $C_3$-$C_6$-Alkinyl bedeuten;

d) bis zu 2 der Substituenten $R^1$ bis $R^6$, die nicht benachbart sein sollen, die folgenden Gruppen:
Aziridinyl, Pyrrolidino, Piperidino oder Morpholino, wobei die beiden letztgenannten Reste bis zu zwei der folgenden Substituenten tragen können: $C_1$-$C_{10}$-Alkyl, $C_2$-$C_{10}$-Alkoxyalkyl, $C_2$-$C_{10}$-Alkylthioalkyl, Phenyl oder Benzyl;

e) bis zu 2 der Substituenten $R^1$ bis $R^6$, die nicht benachbart sein sollen, die folgenden Gruppen:
Phenyl, Benzyl oder $C_5$-$C_6$-Heteroaryl, an das ein Benzolring annelliert sein kann, wobei die Arylreste bis zu 3 der folgenden Reste tragen können: Halogen, Nitro, Cyano, Trifluormethyl, Amino, $C_1$-$C_4$-Alkylamino, Di-($C_1$-$C_4$)-alkylamino, $C_1$-$C_4$-Alkoxy, $C_1$-$C_4$-Halogenalkoxy, $C_2$-$C_4$-Alkoxyalkyl, $C_1$-$C_4$-Alkylthio oder $C_1$-$C_4$-Halogenalkylthio;

f) $R^2$, $R^6$ eine Gruppe der allgemeinen Formel:
$YR^9$, wobei Y Sauerstoff, Schwefel, eine Gruppe -O-CO- oder $-NR^8$-und $R^9$ Phenyl oder Benzyl bedeuten, die ein Phenylteil bis zu 3 der folgenden Substituenten tragen können: Halogen, Cyano, Nitro oder Trifluormethyl;

g) $R^2$, $R^6$:
eine Gruppe der allgemeinen Formel IIa oder IIb

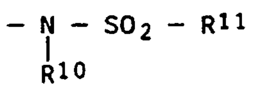

$$-O-SO_2-R^{11}$$

IIa IIb

wobei $R^{10}$ Wasserstoff, $C_1$-$C_{10}$-Alkyl, $C_1$-$C_{10}$-Halogenalkyl, $C_3$-$C_{10}$-Alkenyl, $C_3$-$C_{10}$-Alkinyl, $C_2$-$C_{14}$-Alkoxyalkyl, $C_2$-$C_{14}$-Alkylthioalkyl oder $C_3$-$C_7$-Cycloalkyl bedeutet und $R^{11}$ $C_1$-$C_{10}$-Alkyl, $C_2$-$C_8$-Alkenyl, Trifluormethyl, Cyclopropyl oder eine Phenylgruppe bezeichnet, die ihrerseits bis zu drei der folgenden Substituenten tragen kann: Halogen, Cyano, Nitro, $C_1$-$C_6$-Alkyl oder Trifluormethyl;

h) zwei benachbarte Substituenten der Substituenten $R^1$ bis $R^6$:
zusammen einen annellierten 5- oer 6-gliedrigen ungesättigten Ring, an dessen C-Atomen ein weiterer Benzolring annelliert sein kann und der Schwefel oder bis zu 2 nicht benachbarte Sauerstoff- oder bis zu 3 Stickstoffatome als Heteroatome enthalten kann, wobei diese Reste ihrerseits bis zu 2 der folgenden Substituenten tragen können:
Halogen, Hydroxyl, Nitro, Cyan, Amino, $C_1$-$C_4$-Alkylamino, $C_1$-$C_8$-Dialkylamino, Trifluormethyl, $C_1$-$C_5$-Alkyl, $C_1$-$C_5$-Alkoxy, $C_1$-$C_5$-Halogenalkoxy, $C_1$-$C_5$-Alkylthio, $C_1$-$C_5$-Halogenalkylthio oder Phenyl,

ausgenommen 3,8-Dimethyl-5-isopropyl-1,2-naphthochinon, 3,8-Dimethyl-5-isopropyl-6-hydroxy-1,2-naphthochinon, 1,2-Dihydro-1,5,8-trimethyl-naphtho-[2,1-b]furan-6,7-dion, 2,3-Dihydro-3,6,9-trimethyl-naphtho-[1,8-bc]pyran-7,8-dion und 3,6,9-Trimethylnaphtho-[1,8-bc]pyran7,8-dion.

Durch R.S. Jeng (Experientia 39, 1089 (1983)) sind die fungiziden Eigenschaften von den in der Natur vorkommenden Mansononen, substituierten 1,2-Naphthochinonen, bekannt geworden. Hierbei handelt es sich um ganz spezielle Verbindungen, die in 3- und 8-Stellung Methylgruppen und in 5-Stellung Isopropylgruppen, die mit der Position 4 oder 6 über ein Sauerstoffatom verbunden sein können, tragen. Außerdem sind aus der GB 913 196 fungizide 1,2-Naphthochinonoxime bekannt.

Die in der Natur vorkommenden Mansonone zeigen zwar fungizide Eigenschaften, doch können sie aufgrund ihrer komplexen Struktur nur sehr umständlich und in äußerst geringer Ausbeute aus Extrakten isoliert werden [vgl. R.S. Jeng, Experientia 39, 1089 (1983)].

Der Erfindung lag daher die Aufgabe zugrunde, neue fungizid wirksame Mittel zu finden.

Demgemäß wurden die eingangs definierten fungiziden Mittel gefunden.

Unter den erfindungsgemäßen Mitteln werden diejenigen bevorzugt, in denen die Substituenten der Wirkstoffe I die folgende Bedeutung haben:

a) $R^1$ bis $R^6$: Wasserstoff, Fluor, Chlor, Brom oder Iod;

b) bis zu 3 der Substituenten $R^1$ bis $R^6$:

- Cyan, Nitro;
- $C_1$-$C_{10}$-Alkylgruppen, insbesondere Methyl, Ethyl, n-Propyl, Isopropyl, n-Butyl, Isobutyl, sec.-Butyl, tert.-Butyl, n-Pentyl, 2,2-Dimethylpropyl, Pent-3-yl, 1,2-Dimethylpropyl, n-Hexyl, 1,3-Dimethylbutyl, 1-Ethyl-2-methyl-propyl, 1,2,2-Trimethyl-propyl, 1,2-Dimethylhexyl oder 2-Cyan-1-methoxycarbonyl-methyl;
- $C_1$-$C_{10}$-Hydroxyalkylgruppen, bevorzugt $C_1$-$C_4$-Hydroxyalkylgruppen wie Hydroxymethyl, 2-Hydroxyethyl, 3-Hydroxypropyl, 2-Hydroxy-isopropyl, 2-Hydroxybutyl, 3-Hydroxybutyl, 4-Hydroxybutyl oder 2-Hydroxyisobutyl;
- $C_1$-$C_{10}$-Halogenalkylgruppen, bevorzugt $C_1$-$C_4$-Halogenalkylgruppen wie Fluormethyl, Chlormethyl, Difluormethyl, Dichlormethyl, Trifluormethyl, Trichlormethyl, 2-Chlorethyl, 2-Chlorpropyl, 3-Fluorpropyl, 3-Chlorpropyl, 2-Fluor-isopropyl, 2-Chlor-isopropyl, 2-Fluorethyl, 2,2,2-Trifluorethyl oder Pentafluorethyl;
- $C_2$-$C_{10}$-Alkenylgruppen, insbesondere Allyl, Vinyl, Crotyl, But-1-en-3-yl, 2-Methyl-but-2-enyl, 2-Methyl-but-3-enyl, 2-Methyl-but-1-en-4-yl, 2-Methyl-but-2-en-4-yl, 3-Methyl-but-1-en-3-yl, Hex-5-enyl oder 2-Ethyl-hex-2-enyl;
- $C_2$-$C_{10}$-Halogenalkenylgruppen, bevorzugt $C_2$-$C_4$-Halogenalkenylgruppen wie 3-Chlor-prop-2-en-1-yl;

$C_2$-$C_{10}$-Alkinylgruppen, bevorzugt $C_2$-$C_4$-Alkinylgruppen wie Propinyl, But-2-inyl oder But-1-in-3-yl;

- $C_3$-$C_6$-Cycloalkylgruppen, bevorzugt Cyclopropyl, Cyclopentyl oder Cyclohexyl;
- $C_3$-$C_6$-Alkoxy-alkoxycarbonylgruppen;

c) bis zu 3 der Substituenten $R^1$ bis $R^6$:

- Hydroxyl- und Thiolgruppen;
- $C_1$-$C_4$-Alkoxy-$C_1$-$C_{10}$-alkylgruppen, bevorzugt 2-Methoxyethyl, 2-Ethoxyethyl, 3-Methoxypropyl, 2-Methoxy-isopropyl, 3-Methoxy-butyl, 1-Methoxy-sec.-butyl, 2-Methoxy-butyl, 4-Methoxy-butyl, Methoxy-tert.-butyl, Ethoxy-tert.-butyl oder Methoxymethyl;
- $C_1$-$C_4$-Alkylthio-$C_1$-$C_{10}$-alkylgruppen, bevorzugt Methylthiomethyl oder Methylthioethyl;
- $C_1$-$C_5$-Alkoxygruppen, insbesondere Methoxy, Ethoxy, n-Propoxy, Iso-propoxy, n-Butoxy, iso-Butoxy, tert.-Butoxy, Pentoxy, Difluormethoxy, Trifluormethoxy oder Trichlormethoxy;
- $C_1$-$C_5$-Alkylthiogruppen, insbesondere Methylthio, Ethylthio, n-Propylthio, Isopropylthio, n-Butylthio, iso-Butylthio, tert.-Butylthio, Difluormethylthio oder Trifluormethylthio;
- $C_1$-$C_6$-Alkylcarbonylgruppen, bevorzugt $C_2$-$C_4$-Alkylcarbonylgruppen wie Methylcarbonyl, Trifluormethylcarbonyl oder Trichlormethylcarbonyl;
- $C_2$-$C_6$-Alkoxycarbonylgruppen, insbesondere Methoxycarbonyl, Ethoxycarbonyl, n-Propoxycarbonyl, iso-Propoxycarbonyl, n-Butoxycarbonyl, iso-Butoxycarbonyl, tert.-Butoxycarbonyl, 2-Methoxyethoxycarbonyl, Ethoxy- methoxycarbonyl oder 2-Ethoxy-ethoxycarbonyl;
- $C_2$-$C_6$-Alkanoyloxy- oder $C_2$-$C_6$-Halogenalkanoyloxygruppen, insbesondere Acetyl, Propionyl, Butyryl, Isobutyryl, Valeryl, Isovaleryl, Pivalyl, Fluoracetyl, Difluoracetyl, Trifluoracetyl, Chloracetyl, Dichloracetyl, Trichloracetyl, 2-Fluorpropionyl, 2-Chlor-propionyl, 2,2-Difluorpropionyl, 2,2-Dichlorpropionyl, 3,3,3-Trifluorpropionyl oder Pentafluorpropionyl;
- Aminogruppen oder gewünschtenfalls halogenierte $C_1$-$C_5$-Alkylamino- oder Di-($C_1$-$C_5$)-alkylaminogruppen, bevorzugt $C_1$-$C_4$-Alkylamino- oder Di-($C_1$-$C_4$)-alkylaminogruppen wie Methylamino, Ethylamino, Isopropylamino, n-Butylamino, iso-Butylamino, tert.-Butylamino, Dimethylamino, Diethylamino, Diisopropylamino, Methylethylamino oder 2-Chlorethylamino;
- $C_1$-$C_4$ -Alkylcarbonylaminogruppen, $C_1$-$C_4$-Alkylcarbonylamino-$C_1$-$C_5$-alkylgruppen, $C_1$-$C_5$-Alkylamino-

carbonylgruppen, $C_1$-$C_5$-Halogenaminocarbonylgruppen oder Di-($C_1$-$C_5$)-alkylaminocarbonylgruppen, insbesondere Acetylamino oder Dimethylaminocarbonyl;

d) bis zu 2 der Substituenten $R^1$ bis $R^6$

- Aziridinyl-, Pyrrolidino- oder gewünschtenfalls substituierte Piperidino- oder Morpholinogruppen, insbesondere 1-Piperidino, 1-(4-(4-tert.-Butylphenyl)-piperidino), 4-Morpholino oder 4-(cis-2,6-Dimethylmorpholino);

e) bis zu 2 der Substituenten $R^1$ bis $R^6$

- gewünschtenfalls kernsubstituierte Phenyl- oder Benzylgruppen, insbesondere Phenyl, 2-Fluorphenyl, 3-Fluorphenyl, 4-Fluorphenyl, 2-Chlorphenyl, 3-Chlorphenyl, 4-Chlorphenyl, 2,4-Difluorphenyl, 4-Trifluormethylphenyl, 2,4-Di-trifluormethylphenyl, 2-Nitrophenyl, 3-Nitrophenyl, 4-Nitrophenyl, 2-Cyanophenyl, 3-Cyanophenyl, 4-Cyanophenyl, 2-Aminophenyl, 3-Aminophenyl, 4-Aminophenyl, 4-Methylaminophenyl, 4-Dimethylaminophenyl, 2-Methoxyphenyl, 3-Methoxyphenyl, 4-Methoxyphenyl, 2,4-Dimethoxyphenyl, 2,4,5-Trimethoxyphenyl, 2-Methylthiophenyl, 3-Methylthiophenyl, 4-Methylthiophenyl, 4-(Methoxymethyl)-phenyl oder Benzyl;

- gewünschtenfalls substituierte $C_5$-$C_6$-Heteroarylgruppen, insbesondere 2-Pyrrolyl, 2-Furanyl, 3-Furanyl, 2-methyl-furan-5-yl, 2-Thienyl, 3-Thienyl, 2-Methyl-thien-5-yl, 2-Imidazolyl, 2-Pyridyl, 3-Pyridyl, 4-Pyridyl, 3-Chlor-pyrid-6-yl, 4,6-Dimethyl-pyrimid-2-yl oder Benzimidazol-2-yl;

f) $R^2$, $R^6$

- gewünschtenfalls kernsubstiuierte Phenoxy-, Benzyloxy-, Phenylthio- oder Benzylthiogruppen, insbesondere Phenoxy, 4-Fluorphenoxy, 4-Chlorphenoxy, 2,4-Difluorphenoxy, 2,4-Dichlorphenoxy, 2,4,6-Triflurorphenoxy, 2-Cyanphenoxy, 3-Cyanphenoxy, 4-Cyanphenoxy, 4-Cyan-2,6-dibromphenoxy, 4-Cyan-2-methoxy-phenoxy, 2-Nitrophenoxy, 3-Nitrophenoxy, 4-Nitrophenoxy, 4-Trifluormethylphenoxy, 2-Chlor-4-trifluormethylphenoxy, Benzyloxy, 2-Fluorbenzyloxy, 3-Fluorbenzyloxy, 4-Fluorbenzyloxy, 2-Chlorbenzyloxy, 3-Chlorbenzyloxy, 4-Chlorbenzyloxy, 2,4-Difluorbenzyloxy, 2,4-Dichlorbenzyloxy, 4-Cyan-benzyloxy, 4-Nitro-benzyloxy, 4-Trifluormethylbenzyloxy, 2-Chlor-6-trifluormethylbenzyloxy, Phenylthio, 2-Fluorphenylthio, 3-Fluorphenylthio, 4-Fluorphenylthio, 4-Chlorphenylthio, 2,3,4,5-Tetrafluor-phenylthio, 4-Methylphenylthio, 4-Trifluormethyl-phenylthio, 2-Cyan-phenylthio, 3-Cyan-phenylthio, 4-Cyan-phenylthio oder Benzylthio;

- gewünschtenfalls kernsubstituierte Benzoyloxy- oder Benzylcarbonyloxygruppe, insbesondere Benzoyloxy, 2-Fluorbenzoyloxy, 3-Fluorbenzoyloxy, 4-Fluorbenzoyloxy, 2-Chlorbenzoyloxy, 3-Chlorbenzoyloxy, 4-Chlorbenzoyloxy, 1,3,5-Trichlorbenzoyloxy, p-Cyanobenzoyloxy, 2,4-Dinitrobenzoyloxy, 4-Trifluormethy-lbenzoyloxy oder Benzylcarbonyloxy;

- gewünschtenfalls Phenyl- oder Benzylaminogruppe, insbesondere Phenylamino oder Benzylamino;

g) $R^2$, $R^6$

- gewünschtenfalls substituierte Sulfonat- oder Sulfonamidgruppen, wobei der Schwefel mit $C_1$-$C_{10}$-Alkyl, Trifluormethyl oder Phenyl substituiert ist, insbesondere Methylsulfonat, Ethylsulfonat, Propylsulfonat, Trifluormethylsulfonat, Benzolsulfonat, p-Toluolsulfonat, 2,5-Dichlorbenzolsulfonat, Pentafluorbenzolsulfonat, 4-Cyan-benzolsulfonat, 4-Nitrobenzolsulfonat, 4-Trifluormethylbenzolsulfonat, Methylsulfonamido, Trifluormethylbenzolsulfonamido, N-Methyl-methylsulfonamido, 2,6-Difluorbenzolsulfonamido, 2-Chlorbenzolsulfonamido, p-Toluolsulfonamido, N-Methyl-p-toluolsulfonamido, 4-Trifluormethyl-benzolsulfonamido, N-Allyl-2,6-Difluorbenzolsulfonamido oder N-Cyclopropyl-2-Chlorbenzolsulfonamido;

h) zwei benachbarte Substituenten der Substituenten $R^1$ bis $R^6$ zusammen einen 5- oder 6-gliedrigen ungesättigten Ring, an den ein Benzolring annelliert sein kann und der noch 1-2 der folgenden Substituenten tragen kann:

- Halogen, insbesondere Fluor, Chlor oder Brom,
- Hydroxyl,
- Nitro,
- Cyan,
- Amino, $C_1$-$C_4$-Alkylamino oder $C_2$-$C_8$-Dialkylamino,
- Trifluormethyl,
- $C_1$-$C_5$-Alkyl, insbesondere Methyl, Ethyl, Propyl oder Isopropyl,
- $C_1$-$C_5$-Alkoxy, insbesondere Methoxy, Ethoxy, Propoxy oder Isopropoxy,
- $C_1$-$C_5$-Halogenalkoxy, bevorzugt $C_1$-$C_3$-Halogenalkoxy,
- $C_1$-$C_5$-Alkylthio, insbesondere Methylthio, Ethylthio, Propylthio oder Isopropylthio,
- $C_1$-$C_5$-Halogenalkylthio, bevorzugt $C_1$-$C_3$-Halogenalkylthio.

Bevorzugte annellierte Ringsysteme sind Thiophen, Furan, 2-Isopropylfuran, Dihydrofuran, 3-Methyl-1,2-Dihydrofuran, 2-Isopropyl-3-ethoxy-1,2-dihydrofuran, 1,3-Dioxolan, 2,2-Dimethyl-1,3-dioxolan, 2,2-Diphenyl-1,3-dioxolan, 2-Oxo-1,3-dioxolan, Pyrazol, 1-Methylpyrazol, Triazol, Indol, 1-Methylindol, Benzol, 1,4-Dioxan,

Tetrahydropyrazin oder N,N′-Dimethyl-tetrahydropyrazin.

Besonders gut geeignete Verbindungen sind in Tabelle 1 unter den Wirkstoffbeispielen aufgeführt.

Die 1,2-Naphthochinone der Formel I sind bekannt oder lassen sich nach verschieden bekannten Verfahren darstellen, wie sie in Houben-Weyl (Methoden der organischen Chemie, Band 7/3b, S. 1ff. (1979)) oder von K.T. Finley (in S. Patai, The Chemistry of Chinoid Compounds Part II, p. 877ff. (1974)) eingehend beschrieben werden.

Im folgenden sind einige Verfahren zur Darstellung der 1,2-Naphthochinone I beispielhaff aufgeführt:

A) Oxidation von o-Halogennaphtholen

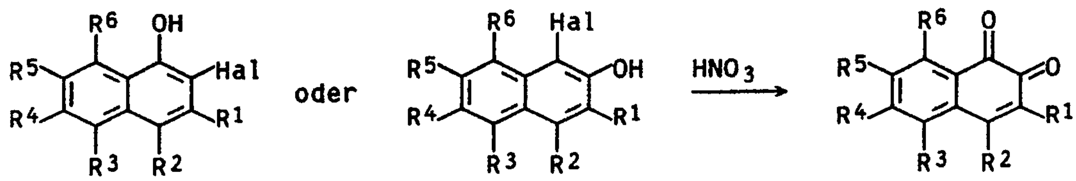


IIa IIb I

In den Formeln IIa und IIb bedeutet Hal ein Halogenatom, insbesondere Chlor oder Brom.

Die Reaktion wird mit oxidierenden Säuren, bevorzugt Salpetersäure, in einem inerten Lösungsmittel wie Essigsäure oder auch in Lösungsmittelgemischen wie Essigsäure/Wasser, durchgeführt. Im allgemeinen verläuft die Oxidation bei Temperaturen zwischen 0° C und dem Siedepunkt des Lösungsmittels bzw. Lösungsmittelgemisches hinreichend schnell und ist nach ca. 1 bis 12 Stunden beendet. Das Produkt wird beispielsweise durch zugabe von Wasser ausgefällt.

Die Säure wird vorteilhaft in der doppelten molaren Menge, bezogen auf das Edukt, eingesetzt. Höhere oder niedrigere Mengen sind auch möglich, bringen jedoch keine Vorteile.

Da die Oxidation nicht druckabhängig ist, arbeitet man vorzugsweise bei Normaldruck.

Die Naphthole IIa und IIb sind handelsüblich oder können nach bekannten Methoden hergestellt werden.

B) Oxidation von Naphtholen

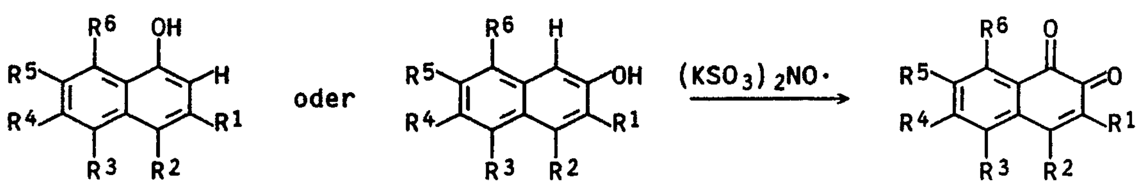


IIIa IIIb I

Die Oxidation von Naphtholen IIIa und IIIb mit oxidierenden Salzen, z.B. mit Fremy's Salz ($[KSO_3]_2NO^\bullet$), ist ein besonders bevorzugtes Verfahren zur Herstellung der 1,2-Naphthochinone I.

Variante 1):

Die Oxidation wird mit einer wäßrigen Lösung des Oxidationssalzes bei einer Temperatur von z.B. (-10) bis 20° C, bevorzugt Raumtemperatur, durchgeführt. Für die Naphthole IIIa und IIIb empfehlen sich inerte Lösungsmittel wie Methanol, Ethanol, Isopropanol oder Aceton. Das Produkt wird z.B. durch zugabe von Wasser ausgefällt.

Variante 2):

Die Oxidation wird in einem Zweiphasen-System bei einer Temperatur von z.B. (-5) bis 50 °C, bevorzugt 0 bis 30 °C, insbesondere 20 °C, durchgeführt, wobei das Oxidationssalz in wäßriger Lösung vorliegt. Für die Naphthole IIIa und IIIb empfehlen sich dabei inerte Lösungsmittel wie Dichlormethan, Chloroform, Dichlorethan, Diethylether, Methyl-tert.-butylether, Benzol, Toluol oder Xylole.

Besonders vorteilhaft arbeitet man in Gegenwart eines Phasentransferkatalysators wie Tetra-n-butylammoniumbromid oder Benzyl-triethylammoniumchlorid. Das Produkt befindet sich in der organischen Phase.

Bei beiden Varianten arbeitet man in einem gepufferten System (pH-Wert = 6-7) und verwendet bevorzugt einen Überschuß an Oxidationssalz, z.B. die 2- bis 5-fache, inbesondere die 2- bis 3-fache, molare Menge, bezogen auf die Menge an Naphthol.

Bezüglich der Reaktionsdauer, des Druckes und der Edukte gelten die für Methode A) gemachten Angaben.

C) Oxidation von Dihydroxy- oder Diaminonaphthalinen

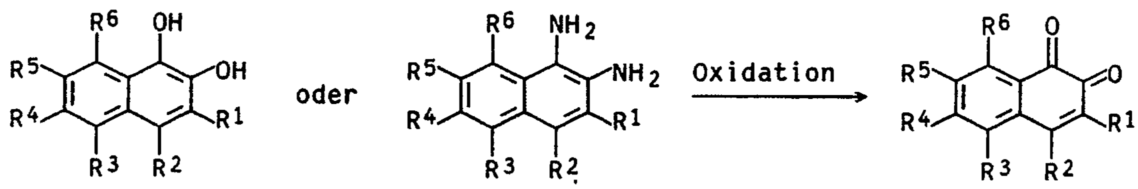


IVa IVb I

Zur Oxidation der Dihydroxynaphthaline IVa verwendet man bevorzugt Blei-(IV)-acetat, Natriumtetraoxoperjodat, Salpetersäure, Silber-(I)-oxid, Silbercarbonat, Eisen-(III)-chlorid, Tetrachlor-o-benzochinon, Dichlor-dicyano-p-benzochinon, Chrom-(VI)-oxid oder Kaliumdichromat in Verbindung mit Schwefelsäure.

Die Oxidation der Diaminonaphthaline IVb erfolgt vorteilhaft mit Eisen-(III)-chlorid.

Für die Naphthaline IVa und IVb empfehlen sich inerte Lösungsmittel wie Nitromethan, Nitroethan, Methanol, Ethanol, Isopropanol, Aceton, Essigsäure oder Wasser.

Vorteilhaft gibt man äquimolare Mengen an Oxidationsmittel zu den Verbindungen IVa und IVb bei Temperaturen von z.B. (-50) bis 80 °C.

Bezüglich der Reaktionsdauer, des Druckes, der Edukte und der Isolierung der Produkte gelten die Angaben für Methode A).

D) Oxidation von Aminonaphtholen

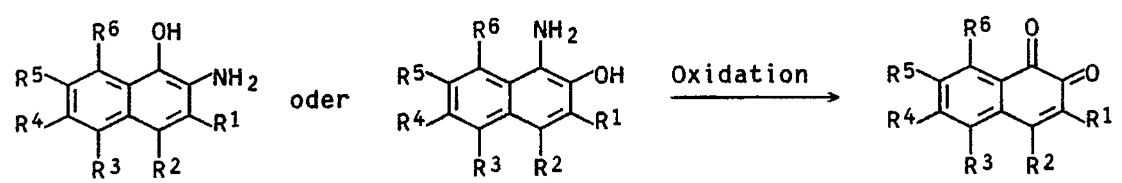


Va Vb I

Die Oxidation der Aminonaphthole erfolgt vorteilhaft mit Eisen-(III)-chlorid, Salpetersäure, Chrom-(VI)-oxid oder Kaliumdichromat in Verbindung mit Schwefelsäure.

Bezüglich des Lösungsmittels, der stöchiometrischen Verhältnisse, der Temperatur, der Reaktionsdauer, des Druckes, der Edukte und der Isolierung der Produkte gelten die Angaben für Methode A).

E) Oxidative Halogenierung von Naphthochinon-sulfonsäuren

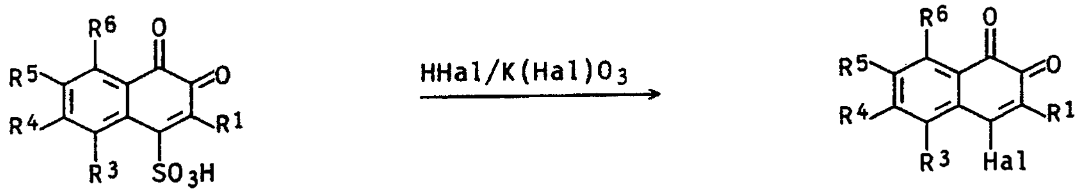


VI

I ($R^2$ = Hal)

Hal bedeutet Halogen, insbesondere Chlor oder Brom.

Bevorzugt versetzt man eine Lösung des Naphthochinons VI in einem inerten Solvens wie Wasser mit einer wäßrigen Lösung der Kaliumhalogenate in den entsprechenden Halogenwasserstoffsäuren. Vorteilhaft verwendet man äquimolare Mengen an Halogenaten, oder einen geringen Überschuß bis etwa 10 %, bei Reaktionstemperaturen von beispielsweise (-5) bis 80 °C. Das Produkt wird nach üblichen Methoden isoliert.

Bezüglich der Reaktionsdauer, des Druckes und der Edukte gelten die Angaben für Methode A).

F) Halogenierung von Naphthochinonen

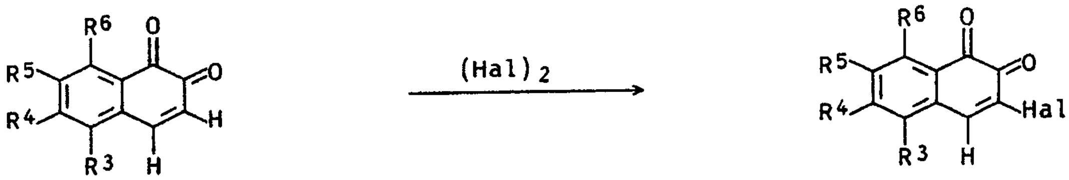


I ($R^1$, $R^2$ = H)

I ($R^1$ = Hal, $R^2$ = H)

Bevorzugt versetzt man eine Lösung des Naphthochinons I ($R^1$, $R^2$ = H) in einem inerten Lösungsmittel wie Essigsäure oder einem Gemisch aus Essigsäure und Wasser mit dem Halogen.

Vorteilhaft verwendet man einen Überschuß an Halogen, z.B. 10 bis 150 %, bei einer Reaktionstemperatur von beispielsweise 0 bis 150 °C, insbesondere 20 bis 120 °C. Das Produkt wird wie üblich isoliert.

Bezüglich der Reaktionsdauer, des Druckes und der Edukte gelten die Angaben für Methode A).

G) Oxidative Aminierung von Naphthochinonen

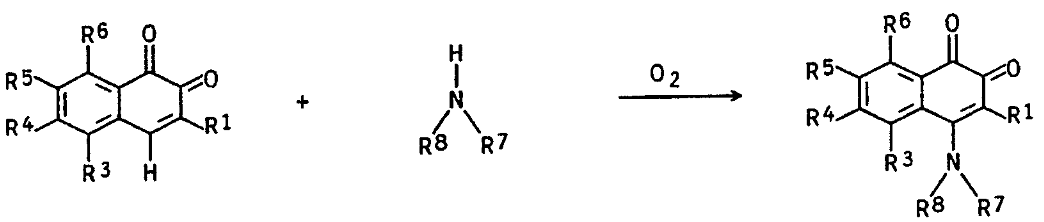


I ($R^2$ = H)

I ($R^2$ = $-NR^7R^8$)

Bevorzugt versetzt man eine Lösung des Naphthochinons I ($R^2$ = H) in einem inerten Lösungsmittel wie Methanol, Ethanol, Propanol, Isopropanol oder n-Butanol mit dem Amin.

Vorteilhaft verwendet man äquimolare Mengen an Naphthochinon und Amin bei einer Reaktionstemperatur von beispielsweise (-10) bis 120 °C, insbesondere 0 bis 90 °C. Die Reaktion wird in Gegenwart eines oxidationsmittels, besonders bevorzugt Sauerstoff, durchgeführt. Das Produkt wird wie üblich isoliert.

Bezüglich der Reaktionsdauer, des Druckes und der Edukte gelten die Angaben für Methode A).

H) Alkylierung von Salzen der 2-Hydroxy-1,4-naphthochinone

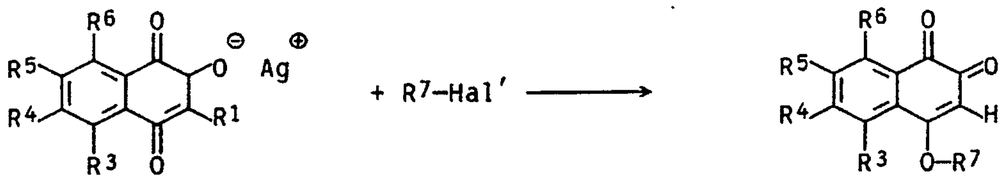

VII I ($R^1$ = H, $R^2$ = $OR^7$)

Hal′ bedeutet Halogen wie Chlor, Brom oder Iod, besonders bevorzugt Brom oder Iod.

Bevorzugt versetzt man eine Suspension des Silbersalzes VII in einem inerten Lösungsmittel wie Dichlormethan, Chloroform, Dichlorethan, Diethylether, Methyl-tert.-butylether, Benzol, Toluol, oder Xylole mit dem Alkylierungsmittel $R^7$-X. Das Alkylierungsmittel kann auch in einem der genannten Solventien gelöst werden.

Vorteilhaft verwendet man einen geringen Überschuß an Halogenalkyl bis etwa 20 %, bezogen auf die Menge an p-Naphthochinon-Salz, bei einer Reaktionstemperatur von beispielsweise (-30) bis 150 °C, besonders bevorzugt bei der Rückflußtemperatur des verwendeten Lösungsmittels oder Lösungsmittelgemisches. Das Produkt wird wie üblich isoliert.

Bezüglich der Reaktionsdauer und des Druckes gelten die Angaben für Methode A).

Die als Ausgangsmaterialien benötigten Silbersalze VII können nach literaturbeschriebenen Verfahren aus den bekannten 2-Hydroxy-1,4-naphthochinonen [vgl. J. Org. Chem. 53, 808 (1988) und J. Am. Chem. Soc. 100, 6728 (1978)] dargestellt werden. Die Halogenalkyle sind entweder handelsüblich oder können nach literaturbekannten Verfahren hergestellt werden.

Die Verbindungen I eignen sich als Fungizide.

## Herstellungsbeispiele

### Beispiel 1 (Verfahrensvariante H)

4-Methoxy-1,2-naphthochinon (Verbindung 1 in Tabelle 1)

Eine Suspension von 60,4 g (0,26 mol) Silberoxid in 200 ml Chloroform wurde mit 17,4 g (0,1 mol) 2-Hydroxy-1,4-naphthochinon und anschließend tropfenweise mit 17,0 g (0,12 mol) Methyliodid versetzt. Danach wurde 4 Stunden zum Rückfluß erhitzt und wie üblich auf das Produkt hin aufgearbeitet. Zur Reinigung wurde das Rohprodukt in 500 ml gesättigter Natriumhydrogensulfitlösung eingerührt und mit gesättigter Natriumcarbonatlösung wieder ausgefällt. Die Umkristallisation aus Toluol lieferte das gelbe Produkt in 45 %ger Ausbeute.

### Beispiel 2 (Verfahrensvariante G)

4-[4-(4-tert.Butylphenyl)-1-piperidinyl]-1,2-naphthochinon (Verbindung 2 in Tabelle 1)

Eine Mischung aus 3,2 g (0,02 mol) 1,2-Naphthochinon in 150 ml Isopropanol und 4,4 g (0,02 mol) 4-(4-tert.-Butylphenyl)-piperidin in 50 ml Isopropanol wurde bis zur Beendigung der Sauerstoffaufnahme in einer Sauerstoffatmosphäre gerührt und das ausgefallene rote, neue Produkt mit tert.-Butyl-methylether gewaschen. Ausbeute: 65 %.

### Beispiel 3 (Verfahrensvariante B)

4,8-Dimethoxy-1,2-naphthochinon (Verbindung 3 in Tabelle 1)

Eine Lösung aus 4,0 g (19,6 mmol) 4,8-Dimethoxy-1-naphthol und 0,1 g Tetrabutylammoniumbromid in 150 ml Dichlormethan wurde mit 15,0 g (56 mmol) Kaliumnitrosodisulfonat (Fremy's Salz) in 150 ml 15 %ger Sodalösung versetzt und die entstandene Mischung 3 Stunden bei 20 °C gerührt. Danach wurde die organische Phase wie üblich auf das Produkt hin aufgearbeitet. Die Umkristallisation aus tert.-Butylmethylether lieferte das Produkt in 96 % Ausbeute.

$^1$H-NMR (360 MHz, in $CDCl_3$, TMS als Standard): $\delta$ = 3,98 ppm (s; 3H), 4,00 ppm (s; 3H), 5,95 ppm (s; 1H), 7,18 ppm (d, J = 8Hz; 1H), 7,55 ppm (d, J = 8Hz; 1H), 7,65 ppm (t, J = 8Hz; 1H).

Analog den Verfahrensvarianten A - H oder den in Houben-Weyl, Band 7/3b, S. 1 ff. (1979) beschriebenen Methoden wurden die in Tabelle 1 aufgeführten Wirkstoffe hergestellt.

Tabelle 1

Fungizide 1,2-Naphthochinone

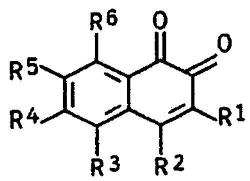

| Wirkstoff Nr. | $R^1$ | $R^2$ | $R^3$ | $R^4$ | $R^5$ | $R^6$ | Fp. [°C] |
|---|---|---|---|---|---|---|---|
| 1 | H | O-Me | H | H | H | H | 192 |
| 2 | H | 4-(4-t.-But-phenyl)-piperidinyl | H | H | H | H | 222 |
| 3 | H | O-Me | H | H | H | O-Me | 203-205 |
| 4 | F | H | H | H | H | H | |
| 5 | F | F | H | H | H | H | |
| 6 | H | F | H | H | H | H | |
| 7 | H | F | H | H | H | F | |
| 8 | H | H | H | H | H | F | |
| 9 | F | F | F | F | F | F | 141 |
| 10 | Cl | H | H | H | H | H | 172 |
| 11 | H | Cl | H | H | H | H | 134 |
| 12 | H | H | H | H | H | Cl | |
| 13 | H | Cl | H | H | H | Cl | |
| 14 | Cl | Cl | H | H | H | H | 184 |
| 15 | H | H | Cl | H | H | Cl | 196 |
| 16 | Cl | Cl | Cl | Cl | Cl | Cl | 145 |
| 17 | Br | Br | H | H | H | H | 173 |
| 18 | H | Br | H | H | H | H | 154 |
| 19 | H | H | Br | H | H | H | 179 |
| 20 | H | H | H | Br | H | H | 168 |
| 21 | H | H | H | H | H | Br | |
| 22 | H | H | H | H | Br | H | |
| 23 | Br | H | H | Br | H | H | 176 |
| 24 | Br | Br | H | Br | H | H | 191 |
| 25 | Br | H | Br | Br | H | H | 184 |

Tabelle 1 (Forts.)

| Wirkstoff Nr. | $R^1$ | $R^2$ | $R^3$ | $R^4$ | $R^5$ | $R^6$ | Fp. [°C] |
|---|---|---|---|---|---|---|---|
| 26 | H | Br | H | H | H | Br | |
| 27 | Cl | H | Br | H | H | H | 177 |
| 28 | H | I | H | H | H | H | |
| 29 | H | I | H | H | H | I | |
| 30 | I | H | H | H | H | H | |
| 31 | $NO_2$ | H | H | H | H | H | 157 |
| 32 | H | $NO_2$ | H | H | H | H | |
| 33 | H | H | H | H | H | $NO_2$ | |
| 34 | H | $NO_2$ | H | H | H | $NO_2$ | |
| 35 | $NO_2$ | Cl | H | H | H | H | 184 |
| 36 | $NO_2$ | H | H | $NO_2$ | H | Cl | 230 |
| 37 | H | $NO_2$ | H | $NO_2$ | H | Cl | |
| 38 | Cl | H | H | H | H | $NO_2$ | |
| 39 | $NO_2$ | H | H | H | O-Me | $NO_2$ | 262 |
| 40 | CN | H | H | H | H | H | |
| 41 | H | CN | H | H | H | H | |
| 42 | H | H | H | H | H | CN | |
| 43 | H | CN | H | H | H | CN | |
| 44 | CN | CN | H | H | H | H | |
| 45 | CN | H | H | H | H | CN | |
| 46 | OH | H | H | H | H | H | |
| 47 | H | OH | H | H | H | H | |
| 48 | H | H | OH | H | H | H | 181 |
| 49 | H | H | H | OH | H | H | 168 |
| 50 | H | H | H | H | OH | H | 198 |
| 51 | H | H | H | H | H | OH | |
| 52 | Cl | H | H | H | H | OH | |
| 53 | Me | H | n-Prop | OH | H | Me | |
| 54 | H | O-Me | OH | H | H | H | |
| 55 | Me | O-Me | H | H | H | OH | |
| 56 | H | O-Me | H | H | H | OH | 235 |
| 57 | H | Cl | H | H | H | OH | |
| 58 | H | H | Cl | H | H | OH | |
| 59 | Cl | H | H | H | H | OH | |
| 60 | Cl | $NH_2$ | H | H | H | H | 260 |
| 61 | H | H | H | H | H | $NH_2$ | |
| 62 | H | $NH_2$ | H | H | H | H | |
| 63 | H | $NH_2$ | H | H | H | $NH_2$ | |

Tabelle 1 (Forts.)

| Wirkstoff Nr. | $R^1$ | $R^2$ | $R^3$ | $R^4$ | $R^5$ | $R^6$ | Fp. [°C] |
|---|---|---|---|---|---|---|---|
| 64 | Me | $NH_2$ | H | H | H | H | |
| 65 | Me | H | H | H | H | $NH_2$ | |
| 66 | Me | $NH_2$ | H | H | H | $NH_2$ | |
| 67 | Me | H | H | H | H | H | 122 |
| 68 | H | Me | H | H | H | H | 114 |
| 69 | H | H | Me | H | H | H | 157 |
| 70 | H | H | H | Me | H | H | |
| 71 | H | H | H | H | Me | H | |
| 72 | H | H | H | H | H | Me | |
| 73 | Me | H | H | H | Me | H | 151 |
| 74 | H | Me | H | H | H | OH | |
| 75 | H | H | Me | H | H | OH | |
| 76 | H | Et | H | H | H | H | 109-112 |
| 77 | H | Et | H | H | H | Et | |
| 78 | H | H | H | H | H | Et | |
| 79 | Me | Me | H | H | H | $CH_3$ | 134 |
| 80 | H | i-Prop | H | H | H | H | |
| 81 | H | i-Prop | H | H | H | i-Prop | |
| 82 | H | H | H | H | H | i-Prop | |
| 83 | H | H | i-Prop | H | H | H | 97 |
| 84 | H | t-But | H | H | H | H | |
| 85 | H | H | H | H | H | t-But | |
| 86 | H | t-But | H | H | H | t-But | |
| 87 | t-But | H | H | t-But | H | H | 136 |
| 88 | Allyl | H | H | H | H | H | |
| 89 | H | Allyl | H | H | H | H | |
| 90 | H | H | H | H | H | Allyl | |
| 91 | H | $CH_2$-CH=CHCl | H | H | H | H | |
| 92 | H | H | H | H | H | $CH_2$-CH=CHCl | |
| 93 | H | $CH_2$-C≡CH | H | H | H | H | |
| 94 | H | H | H | H | H | $CH_2$-C≡CH | |
| 95 | $CF_3$ | H | H | H | H | H | |
| 96 | H | $CF_3$ | H | H | H | H | |
| 97 | H | H | H | H | H | $CF_3$ | |
| 98 | H | $CH_2OH$ | H | H | H | H | |
| 99 | H | H | H | H | H | $CH_2OH$ | |

Tabelle 1 (Forts.)

| Wirkstoff Nr. | $R^1$ | $R^2$ | $R^3$ | $R^4$ | $R^5$ | $R^6$ | Fp. [°C] |
|---|---|---|---|---|---|---|---|
| 100 | H | $CH_2OH$ | H | H | H | $CH_2OH$ | |
| 101 | H | Et | H | H | H | $CH_2$-O-Me | |
| 102 | H | $CH_2S$-Me | H | H | H | H | |
| 103 | H | $CH_2$-S-Me | H | H | H | $CH_2$-S-Me | |
| 104 | H | Cyclopropyl | H | H | H | H | |
| 105 | H | Cyclopropyl | H | H | H | Cyclopropyl | |
| 106 | H | H | H | H | H | Cyclopropyl | |
| 107 | H | Morpholino | H | H | H | H | 195 |
| 108 | H | 2,6-$(Me)_2$-morpholino | H | H | H | H | 184 |
| 109 | H | H | H | H | H | 2,6-$(Me)_2$-morpholino | |
| 110 | H | Piperidino | H | H | H | H | |
| 111 | H | H | H | H | H | Piperidono | |
| 112 | H | -S-Ph | H | H | H | H | 168 |
| 113 | H | H | H | H | H | S-Ph | |
| 114 | H | S-Ph(4-Cl) | H | H | H | H | 198 |
| 115 | H | H | H | H | H | S-Ph(4-Cl) | |
| 116 | H | S-Ph(4-Me) | H | H | H | H | 204 |
| 117 | H | O-Ph | H | H | H | H | 168 |
| 118 | H | H | H | H | H | O-Ph | |
| 119 | H | O-Ph(4-Cl) | H | H | H | H | 179 |
| 120 | H | O-Ph(4-F) | H | H | H | H | |
| 121 | H | O-Ph(4-$CF_3$) | H | H | H | H | |
| 122 | H | H | H | H | H | O-Ph(4-$CF_3$) | |
| 123 | H | NH-Ph | H | H | H | H | 248 |
| 124 | H | NH-Me | H | H | H | H | 248 |
| 125 | H | H | H | H | H | NH-Me | |
| 125 | H | H | H | H | H | NH-Me | |
| 126 | H | NH-But | H | H | H | H | 229 |
| 127 | H | NH-t-But | H | H | H | H | |
| 128 | H | H | H | H | H | NH-t-But | |
| 129 | H | NH-Benzyl | H | H | H | H | 206 |
| 130 | H | H | H | H | H | NH-Benzyl | |
| 131 | H | $N(Et)_2$ | H | H | H | H | 133 |
| 132 | H | H | H | H | H | $N(Et)_2$ | |
| 133 | H | HN-$(CH_2)_2$Cl | H | H | H | H | 198 |
| 134 | Me | H | i-Prop | O-Me | H | Me | 161 |
| 135 | H | H | O-Me | H | H | H | 210 |

Tabelle 1 (Forts.)

| Wirkstoff Nr. | $R^1$ | $R^2$ | $R^3$ | $R^4$ | $R^5$ | $R^6$ | Fp. [°C] |
|---|---|---|---|---|---|---|---|
| 136 | O-Me | H | H | H | H | H | |
| 137 | O-Me | Br | H | H | H | $NO_2$ | 196 |
| 138 | H | O-Me | H | H | H | H | 191 |
| 139 | H | H | H | O-Me | H | H | 142 |
| 140 | H | H | H | H | O-Me | H | 170-173 |
| 141 | H | H | H | H | H | O-Me | |
| 142 | O-Me | H | H | H | H | O-Me | 195 |
| 143 | H | O-Me | H | H | H | O-Me | 200 |
| 144 | H | H | O-Me | H | H | O-Me | 169 |
| 145 | H | O-Me | O-Me | H | H | H | 140 |
| 146 | H | H | O-Me | O-Me | H | H | 171 |
| 147 | CO-Me | O-Me | H | H | H | H | 166 |
| 148 | H | O-Me | H | H | H | CO-Me | |
| 149 | H | O-Me | H | H | H | F | |
| 150 | H | O-i-Prop | H | H | H | H | 126 |
| 151 | Cl | O-Et | H | H | H | H | 149 |
| 152 | Br | H | H | H | O-Me | Cl | 260 |
| 153 | OH | Benzyl | H | H | H | H | 189 |
| 154 | H | H | Cl | Cl | O-Me | O-Me | |
| 155 | Cl | Cl | Cl | O-Me | H | H | 174 |
| 156 | H | H | H | H | O-Me | Cl | 216 |
| 157 | H | H | H | H | O-Et | Cl | 220 |
| 158 | Cl | H | H | H | H | O-Me | |
| 159 | H | Cl | H | H | H | O-Me | |
| 160 | $O-CF_3$ | H | H | H | H | H | |
| 161 | H | $O-CF_3$ | H | H | H | H | |
| 162 | H | H | H | H | H | $O-CF_3$ | |
| 163 | H | $O-CHF_2$ | H | H | H | H | |
| 164 | H | $O-CHF_2$ | H | H | H | $O-CHF_2$ | |
| 165 | H | H | H | H | H | $O-CHF_2$ | |
| 166 | H | S-Me | H | H | H | H | 196 |
| 167 | H | S-Et | H | H | H | H | 130 |
| 168 | H | S-But | H | H | H | H | 116 |
| 169 | H | H | H | H | H | S-Me | |
| 170 | H | S-Me | H | H | H | S-Me | |
| 171 | H | $S-CF_3$ | H | Br | H | H | |
| 172 | $S-CF_3$ | H | H | H | H | H | |
| 173 | H | $S-CF_3$ | H | H | H | $S-CF_3$ | |

Tabelle 1 (Forts.)

| Wirkstoff Nr. | $R^1$ | $R^2$ | $R^3$ | $R^4$ | $R^5$ | $R^6$ | Fp. [°C] |
|---|---|---|---|---|---|---|---|
| 174 | H | H | H | H | H | $S-CF_3$ | |
| 175 | COO-Me | H | H | H | H | H | 110-115 |
| 176 | H | H | H | H | H | COO-Me | |
| 177 | H | COO-Me | H | H | H | H | |
| 178 | H | COOH | H | H | H | H | 164 |
| 179 | H | H | H | H | H | COOH | 215 |
| 180 | COOH | H | H | H | H | H | 154 |
| 181 | CO-Me | H | H | H | H | H | |
| 182 | H | CO-Me | H | H | H | H | |
| 183 | H | H | H | H | H | CO-Me | |
| 184 | $CO-CCl_3$ | H | H | H | H | H | |
| 185 | H | $CO-CCl_3$ | H | H | H | H | |
| 186 | H | H | H | H | H | $CO-CCl_3$ | |
| 187 | $CO-CF_3$ | H | H | H | H | $CO-CF_3$ | |
| 188 | H | NH-CO-Me | H | H | H | H | 260 |
| 189 | NH-CO-Me | H | H | H | H | H | 215 |
| 190 | H | H | H | H | H | NH-CO-Me | |
| 191 | H | NH-CO-Me | H | H | H | NH-CO-Me | |
| 192 | H | $CO-N(Me)_2$ | H | H | H | H | |
| 193 | H | H | H | H | H | $CO-N(Me)_2$ | |
| 194 | Cl | NH-Benzyl | H | H | H | H | 253 |
| 195 | Cl | Cl | H | H | H | NH-Benzyl | |
| 196 | Ph | H | H | H | H | H | |
| 197 | H | Ph | H | H | H | H | 120 |
| 198 | H | H | Ph | H | H | H | 170 |
| 199 | H | H | H | Ph | H | H | |
| 200 | H | H | H | H | Ph | H | |
| 201 | H | H | H | H | H | Ph | |
| 202 | H | H | O-CO-Me | O-CO-Me | H | H | 140 |
| 203 | H | H | H | NH-CO-Me | H | H | 220 |
| 204 | H | 2-F-Phenyl | H | H | H | H | |
| 205 | H | 2-Cl-Phenyl | H | H | H | H | |
| 206 | Ph | Ph | H | H | H | H | 248 |
| 207 | H | Ph-CH-OMe | H | H | H | H | 134 |
| 208 | H | H | H | O-Benzyl | H | H | 140 |
| 209 | H | O-Benzyl | H | H | H | H | 175 |
| 210 | H | H | H | H | H | O-Benzyl | |
| 211 | H | H | H | H | H | O-Benzyl | |

Tabelle 1 (Forts.)

| Wirkstoff Nr. | $R^1$ | $R^2$ | $R^3$ | $R^4$ | $R^5$ | $R^6$ | Fp. [°C] |
|---|---|---|---|---|---|---|---|
| 212 | H | O-Benzyl | H | H | H | OH | |
| 213 | H | O-Benzyl(4-$CF_3$) | H | H | H | OH | |
| 214 | H | CH(CN)-COOMe | H | H | H | H | 130 |
| 215 | H | O-Me | H | H | H | O-Benzyl | 145 |
| 216 | H | O-CO-Me | H | H | H | OH | |
| 217 | H | OH | H | H | H | O-CO-Me | |
| 218 | H | O-CO-Me | H | H | H | O-CO-Me | |
| 219 | H | OH | H | H | H | O-CO-$CF_3$ | |
| 220 | H | O-CO-$CF_3$ | H | H | H | O-CO-$CF_3$ | |
| 221 | H | O-CO-$CF_3$ | H | H | H | OH | |
| 222 | H | OH | H | H | H | O-CO-Ph | |
| 223 | H | O-Me | H | H | H | O-CO-Ph | 145-147 |
| 224 | H | O-Me | H | H | H | O-CO-Me | |
| 225 | H | O-Me | H | H | H | O-CO-$CF_3$ | |
| 226 | H | O-Me | H | H | H | O-CO-Ph(4-Cl) | |
| 227 | H | O-Me | H | H | H | O-CO-Ph(4-F) | |
| 228 | H | O-CO-Ph | H | H | H | O-Me | |
| 229 | H | O-CO-Ph(4-F) | H | H | H | OH | |
| 230 | H | O-Me | H | H | H | O-$SO_2$-Me | |
| 231 | H | O-Me | H | H | H | O-$SO_2$-$CF_3$ | |
| 232 | H | O-$SO_2$-Me | H | H | H | O-Me | |
| 233 | H | O-$SO_2$-$CF_3$ | H | H | H | O-Me | |
| 234 | H | O-Me | H | H | H | O-$SO_2$-Ph(4-Me) | |
| 235 | H | O-$SO_2$-Ph(4-$CH_3$) | H | H | H | O-Me | |
| 236 | H | NH-$SO_2$-Me | H | H | H | O-Me | |
| 237 | H | O-Me | H | H | H | NH-$SO_2$-Me | |
| 238 | H | O-Me | H | H | H | NH-$SO_2$-$CF_3$ | |
| 239 | H | NMe-$SO_2$-Me | H | H | H | O-Me | |
| 240 | -CH=CH-CH=CH- | | H | H | H | H | 210 |
| 241 | H | H | H | -CH=CH-CH=CH- | | H | |
| 242 | -CH(OEt)-CH(i-Prop)-O- | | H | H | H | H | 91 |
| 243 | H | -CH=CH-CH=CH- | | H | H | H | 204 |
| 244 | H | O-Me | H | H | -CH=CH-CH=CH- | | |
| 245 | -O-$(CH_2)_2$-O- | | H | H | H | H | |
| 246 | H | H | H | -O-$(CH_2)_2$-O- | | OH | |
| 247 | -CH=C(i-Prop)-O- | | H | H | H | H | 95 |
| 248 | -CH(Me)-$CH_2$-O- | | H | H | H | H | 145 |
| 249 | H | O-Me | H | -O-C$(Me)_2$-O- | | H | |
| 250 | -CH=CH-S- | | H | H | H | H | |
| 251 | -CH=CH-S- | | H | H | H | OH | |

Die 1,2-Naphthochinone zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Sie können in die üblichen Formulierungen übergeführt werden, wie Lösungen, Emulsionen, Suspensionen, Stäube, Pulver, Pasten und Granulate. Die Anwendungsformen richten sich nach den Verwendungszwecken; sie sollen in jedem Fall eine feine und gleichmäßige Verteilung des 1,2-Naphtho chinons gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha. Die neuen Verbindungen können auch im Materialschutz eingesetzt werden, z.B. gegen Paecilomyces variotii.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. eine Lösung aus 90 Gew.-Teilen der Verbindung Nr. 1 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 2, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes und 40 Mol

Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 9, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 24, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 35, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 56 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 108, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 166, 10 Gew.-Teilen des Natriumsalzes eines Phenosulfonsäureharnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 214, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die erfindungsgemäßen Mittel können in diesen Anwendungsformen auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums:

Die folgende Liste von Fungiziden, mit denen die erfindungsgemäßen Verbindungen gemeinsam angewendet werden können, soll die Kombinationsmöglichkeiten erläutern, nicht aber einschränken:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat)
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithiolo[4,5-b]-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,

N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-Schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethylfuran-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.Butylphenyl)-2-methylpropyl]-piperidin,
1-[2-(2,4-Dichlorphenyl)-4-ethyl-1,3-dioxolan-2-yl-ethyl]-1H-1,2,4-tria-zol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl )-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie Dodecylguanidinacetat,
3-[3-(3,5-Dimethyl-2-oxycyclohexyl)-2-hydroxyethyl]-glutarimid, Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-fluorphenyl)-methylsilyl)-methyl)-1H-1,2,4-triazol.

## Anwendungsbeispiele

### Beispiel 1

Wirksamkeit gegen Pyrenophora teres

Gerstenkeimlinge der Sorte "Igri" wurden im Zweiblattstadium mit 0,05 %igen wäßrigen Suspensionen, die 80 % Wirkstoff der Wirkstoffe gemäß den Tabellenbeispielen 1, 56 und 166 und 20 % Emulgator in der Trockensubstanz enthielten, tropfnaß gespritzt. Nach 24 Stunden wurden die Pflanzen mit einer Sporensuspension des Pilzes Pyrenophora teres infiziert und für 48 Stunden in eine Klimakammer mit hoher Luftfeuchtigkeit bei 18°C gestellt.

Anschließend wurden die Pflanzen im Gewächshaus bei 20 bis 22°C und 70 % relativer Luftfeuchtigkeit für weitere 5 Tage kultiviert. Danach wurde das Ausmaß des Pilzbefalls beurteilt.

Gegenüber einem Kontrollversuch (keine Behandlung, 80 % Pilzbefall) zeigte sich, daß die behandelten Pflanzen nur einen Pilzbefall von 5-15 % hatten.

Beispiel 2

Wirksamkeit gegen Rebenperonospora

Blätter von Topfreben der Sorte "Müller Thurgau" wurden mit 0,025 %igen wäßrigen Suspensionen, die 80 % Wirkstoff und 20 % Emulgator in der Trockensubstanz enthielten, besprüht. Zur Beurteilung der Wirkungsdauer der 1,2-Naphthochinone wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert und die Pflanzen 48 Stunden in einer wasserdampfgesättigten Kammer bei 24°C aufgestellt. Anschließend wurden die Reben 5 Tage in einem Gewächshaus bei Temperaturen zwischen 20 und 30°C und zur Beschleunigung des Sporangienträgerausbruches abermals für 16 Stunden in der feuchten Kammer kultiviert. Danach erfolgte die Beurteilung des Ausmaßes des Pilzbefalls auf den Blattunterseiten.

Das Ergebnis zeigte gegenüber einem Kontrollversuch (keine Behandlung, 80 % Pilzbefall), daß die Wirkstoffe 2, 56, 166 und 214 bei der Anwendung in Form einer 0,025 gew.-%igen Lösung nur einen Pilzbefall von 5-15 % hatten.

## Ansprüche

1. Fungizide Mittel, enthaltend inerte Trägerstoffe und ein 1,2-Naphthochinon der allgemeinen Formel I

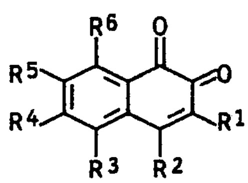

I

in der die Substituenten $R^1$ bis $R^6$ die nachstehende Bedeutung haben:

a) Wasserstoff oder Halogen;

b) bis zu 3 der Substituenten $R^1$ bis $R^6$ die folgenden Gruppen:
Cyan, Nitro, partiell oder vollständig halogeniertes $C_1$-$C_{10}$-Alkyl, $C_2$-$C_{10}$-Alkenyl mit bis zu 2 Halogensubstituenten, $C_2$-$C_{10}$-Alkinyl, $C_3$-$C_6$-Cycloalkyl, $C_3$-$C_6$-Alkoxy-alkoxycarbonyl, $C_1$-$C_{10}$-Alkyl oder $C_2$-$C_{10}$-Alkenyl, wobei die letzten beiden Gruppen bis zu zwei der folgenden Substituenten tragen können: Hydroxy, Cyano, $C_1$-$C_4$-Alkoxy, $C_1$-$C_4$-Alkylthio oder $C_1$-$C_4$-Alkoxycarbonyl;

c) bis zu 3 der Substituenten $R^1$ bis $R^6$ eine Gruppe der allgemeinen Formel:
$XR^7$, wobei X Sauerstoff, Schwefel, eine Gruppe -CO-, -CO-O-, -O-CO-, $-NR^8-$, $-NR^8-CO-$, $-CO-NR^8-$ oder $C_1$-$C_4$-$CO-NR^8$ und $R^7$ und $R^8$ Wasserstoff, $C_1$-$C_5$-Alkyl, das partiell oder vollständig halogeniert sein kann, $C_3$-$C_6$-Alkenyl oder $C_3$-$C_6$-Alkinyl bedeuten;

d) bis zu 2 der Substituenten $R^1$ bis $R^6$, die nicht benachbart sein sollen, die folgenden Gruppen:
Aziridinyl, Pyrrolidino, Piperidino oder Morpholino, wobei die beiden letztgenannten Reste bis zu zwei der folgenden Substituenten tragen können: $C_1$-$C_{10}$-Alkyl, $C_2$-$C_{10}$-Alkoxyalkyl, $C_2$-$C_{10}$-Alkylthioalkyl, Phenyl oder Benzyl;

e) bis zu 2 der Substituenten $R^1$ bis $R^6$, die nicht benachbart sein sollen, die folgenden Gruppen: Phenyl, Benzyl oder $C_5$-$C_6$-Heteroaryl, an das ein Benzolring annelliert sein kann, wobei die Arylreste bis zu 3 der folgenden Reste tragen können: Halogen, Nitro, Cyano, Trifluormethyl, $C_1$-$C_4$-Alkylamino, Di-($C_1$-$C_4$)-alkylamino, $C_1$-$C_4$-Alkoxy, $C_1$-$C_4$-Halogenalkoxy, $C_2$-$C_4$-Alkoxyalkyl, $C_1$-$C_4$-Alkylthio oder $C_1$-$C_4$-Halogenalkylthio;

f) $R^2$, $R^6$ eine Gruppe der allgemeinen Formel: $YR^9$, wobei Y Sauerstoff, Schwefel, eine Gruppe -O-CO- oder -$NR^8$-und $R^9$ Phenyl oder Benzyl bedeuten, die im Phenylrest bis zu 3 der folgenden Substituenten tragen können: Halogen, Cyano, Nitro oder Trifluormethyl;

g) $R^2$, $R^6$:
Gruppen der allgemeinen Formeln IIa oder IIb

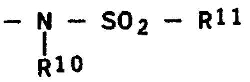

IIa

$$-O-SO_2-R^{11}$$

IIb

wobei $R^{10}$ Wasserstoff, $C_1$-$C_{10}$-Alkyl, $C_1$-$C_{10}$-Halogenalkyl, $C_3$-$C_{10}$-Alkenyl, $C_3$-$C_{10}$-Alkinyl, $C_2$-$C_{14}$-Alkoxyalkyl, $C_2$-$C_{14}$-Alkylthioalkyl oder $C_3$-$C_7$-Cycloalkyl bedeutet und $R^{11}$ $C_1$-$C_{10}$-Alkyl, $C_2$-$C_8$-Alkenyl, Trifluormethyl, Cyclopropyl oder eine Phenylgruppe bezeichnet, die ihrerseits bis zu drei der folgenden Substituenten tragen kann: Halogen, Cyano, Nitro, $C_1$-$C_6$-Alkyl oder Trifluormethyl;

h) zwei benachbarte Substituenten der Substituenten $R^1$ bis $R^6$: zusammen einen annellierten 5- oder 6-gliedrigen ungesättigten Ring, an dessen C-Atomen ein weiterer Benzolring annelliert sein kann und der Schwefel oder bis zu 2 nicht benachbarte Sauerstoff- oder bis zu 3 Stickstoffatome als Heteroatome enthalten kann, wobei diese Reste ihrerseits bis zu 2 der folgenden Substituenten tragen können: Halogen, Hydroxyl, Nitro, Cyano, Amino, $C_1$-$C_4$-Alkylamino, $C_1$-$C_8$-Dialkylamino, Trifluormethyl, $C_1$-$C_5$-Alkyl, $C_1$-$C_5$-Alkoxy, $C_1$-$C_5$-Halogenalkoxy, $C_1$-$C_5$-Alkylthio, $C_1$-$C_5$-Halogenalkylthio oder Phenyl, ausgenommen 3,8-Dimethyl-5-isopropyl-1,2-naphthochinon.

2. Fungizide Mittel, enthaltend inerte Trägerstoffe und ein 1,2-Naphthochinon I nach Anspruch 1, wobei $R^6$ eine Hydroxylgruppe bedeutet.

3. Fungizide Mittel, enthaltend inerte Trägerstoffe und ein 1,2-Naphthochinon I nach Anspruch 2, wobei $R^2$ Fluor, Chlor, Brom eine Trifluormethylgruppe oder eine $C_1$-$C_4$-Alkoxygruppe bedeutet.

4. Fungizide Mittel, enthaltend inerte Trägerstoffe und ein 1,2-Naphthochinon I nach Anspruch 1, wobei $R^2$ für eine Methoxygruppe, eine Methylthiogruppe, eine Gruppe CH(CN)-COOMe oder eine 4-(4-tert.-Butylphenyl)-piperidin-1-yl-gruppe steht und die übrigen Reste Wasserstoff bedeuten.

5. Verfahren zur Bekämpfung von Pilzen, dadurch gekennzeichnet, daß man eine fungizid wirksame Menge eines Mittels, enthaltend ein 1,2-Naphthochinon der Formel I gemäß Anspruch 1, auf Pilze oder durch Pilzbefall bedrohte Materialien, Flächen, Pflanzen oder Saatgut einwirken läßt.

Europäisches Patentamt

# EUROPÄISCHER RECHERCHENBERICHT

Nummer der Anmeldung

EP 90 11 5048

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, Band 69, Nr. 1, 1. Juli 1968, Seite 975, Zusammenfassung Nr. 10301n, Columbus, Ohio, US; B. BABU et al.: "Search for physiologically active compounds. XI. Structure-fungistatic activity relation among substituted 1,2-naphthoquinones", & PROC. INDIAN ACAD. SCI., SECT. A 1967, 66(6), 301-5 * Zusammenfassung * | 1,3,5 | A 01 N 35/06<br>A 01 N 37/34<br>A 01 N 43/16<br>A 01 N 43/40<br>A 01 N 37/02<br>A 01 N 37/10<br>A 01 N 37/22<br>A 01 N 37/18<br>A 01 N 43/12 |
| X | CHEMICA ABSTRACTS, Band 78, Nr. 21, 28. Mai 1973, Seite 84, Zusammenfassung Nr. 132685x, Columbus, Ohio, US; & JP-A-72 43 337 (SHIONOGI AND CO., LTD) 19-12-1972 * Zusammenfassung * | 1,5 | |
| D,A | EXPERIENTIA, Band 39, 1983, Seiten 1089-1090, Birkhäuser Verlag, Basel, CH; M.T. DUMAS et al.: "Isolation and identification of six mansonones from Ulmus americana infected with Ceratocystis ulmi" | | |

RECHERCHIERTE SACHGEBIETE (Int. Cl.5)

A 01 N

Der vorliegende Recherchenbericht wurde für alle Patentansprüche erstellt

| Recherchenort | Abschlußdatum der Recherche | Prüfer |
|---|---|---|
| Den Haag | 21 November 90 | DONOVAN T.M. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument